(19) Europäisches Patentamt
European Patent Office
Office européen des brevets

(11) Publication number: **0 341 204 A2**

(12) **EUROPEAN PATENT APPLICATION**

(21) Application number: **89810305.6**

(22) Date of filing: **24.04.89**

(51) Int. Cl.⁴: **C 07 D 231/52**
G 03 C 7/32, G 03 C 7/38

(30) Priority: **04.05.88 GB 8810460**

(43) Date of publication of application:
**08.11.89 Bulletin 89/45**

(84) Designated Contracting States:
**CH DE FR GB IT LI**

(71) Applicant: **ILFORD Limited**
**Town Lane**
**Mobberley Knutsford Cheshire WA16 7HA (GB)**

(72) Inventor: **Webb, Terence Charles**
**5, Racecourse Road**
**Wilmslow Cheshire SK9 5LF (GB)**

**Long, William Edward**
**56 Buckingham Road**
**Wilmslow Cheshire SK9 5LB (GB)**

**Noxon, Raymond Geoffrey**
**66 Sandown Crescent Cuddington**
**Northwich Cheshire CW8 2QW (GB)**

(74) Representative: **Matthews, Richard Nordan**
**Ilford Limited Patents Department Town Lane Mobberley**
**Nr. Knutsford Cheshire WA16 7HA (GB)**

(54) **Photographic colour coupler.**

(57) There is described a pyrazolone colour coupler of general formula I :

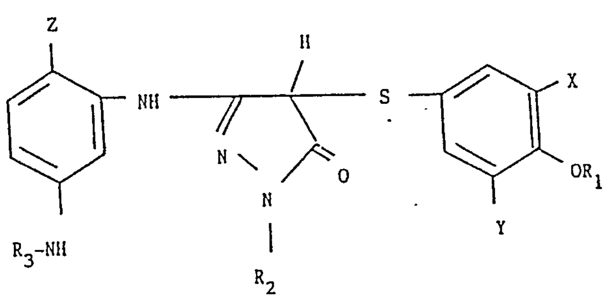

wherein X is a halogen atom, Y and Z are hydrogen, halogen, alkyl, aryl alkoxy, aryloxy, amido or sulphonamido, $R_1$ is an alkyl group having at least 6 carbon atoms, $R_2$ is substituted phenyl and $R_3$ is a long chain ballasting group group which renders the colour coupler oil-soluble.

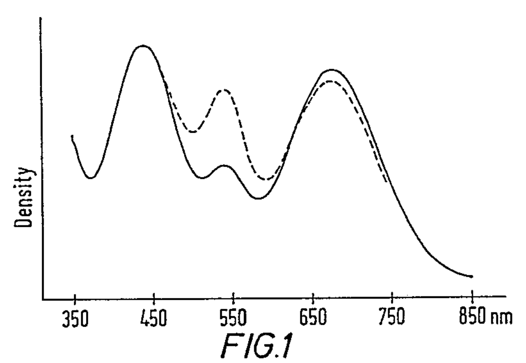

FIG.1

EP 0 341 204 A2

**Description**

## PHOTOGRAPHIC COLOUR COUPLER

This invention relates to novel pyrazolone compounds and to their use in photographic assemblies.

It is well known that pyrazolone compounds can be used as magenta couplers in photographic systems, as described, for example, by L J Fleckenstein in the book The Theory of the Photographic Process edited by T H James, 4th edition, Page 356, 357. The pyrazolone compounds used to date are usually of the type known as four-equivalent couplers. However, it is known that there are disadvantages in the use of such compounds, for not only are four equivalents of silver necessary in order to produce each molecule of dye, but also the couplers themselves are sensitive to certain chemical vapours, for example formaldehyde, and this reduces the achieveable density of magenta dye. These drawbacks can be overcome by the use of so-called two-equivalent pyrazolone couplers in which the coupling site is substituted with a leaving group. Suitable leaving groups for this purpose are known according for example to British patent specification 1 494 777 and 2 086 597 and US patent specification 4556630 but it has proved difficult to prepare and use successfully such 2-equivalent couplers. One of the reasons for this difficulty is that it has been found that the rate-determining step of the coupling reaction of such compounds is the elimination reaction in which the leaving group is expelled, as described by Ichijima and Furutachi in the 1982 International Congress of Photographic Science paper 4 - 18 and by Akhlaq et al in the Journal fur Praktische Chemie, 1987, pages 217 - 223.

This slow elimination step is the limiting factor in the use of such 2-equivalent pyrazolones under certain circumstances. This is particularly true in the case of so-called chromogenic monochrome film, in which a mixture of couplers is used to produce the monochrome image. This is because any delayed production of magenta dye caused by the slow elimination reaction is not only unacceptable in itself, but also causes a concommitant loss of density in the cyan dye, which is located in the same oil droplets. We have found new 2-equivalent pyrazolone magenta colour couplers for which the slow elimination step of the coupling reaction is no longer a limiting factor in their use in photographic assemblies.

Therefore according to the present invention there is provided a pyrazolone colour coupler of general formula I :-

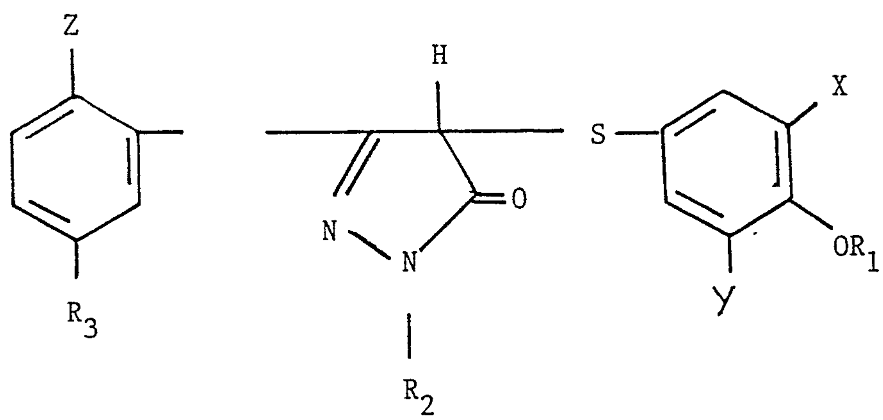

wherein X is a halogen atom, Z and Y are each hydrogen, halogen, alkyl, aryl, alkoxy, aryloxy, amido or sulphonamido, $R_1$ is a alkyl group having at least 6 carbon atoms, $R_2$ is substituted phenyl and $R_3$ is a ballasting group which renders the colour coupler oil-soluble and which is attached to the substituted phenyl ring by a nitrogen atom.

Preferably $R_1$ is a straight or branched chain or cyclic alkyl group having 8 - 12 carbon atoms such as n-octyl, n-nonyl, cyclooctyl, 2-ethylhexyl, trimethylhexyl and cyclododecyl.

Preferably X is a chlorine atom.

Preferably Z is a chlorine atom. If Z is not a halogen atom it is preferably lower alkyl or alkoxy having 1 to 4 carbon atoms. If it is aryl or aryloxy it is preferably phenyl or phenoxy. Suitably the acylamino group has the formula -NHCOR where R is an alkyl group having 1 to 4 carbon atoms. Suitably the sulphonamido groups has the formula $-NHSO_2$ R where R is a alkyl so groups having 1 to 4 carbon atoms.

Preferably Y is a hydrogen atom a chlorine atom or a methyl group. If Y is not a hydrogen atom, a chlorine atom or a methyl groups it is preferably an aryl, aryloxy, alkoxy, acylamino or sulphonamido group as just specifi ed.

Preferably the phenyl group $R_2$ is substituted in the 2, 4 and 6 positions. Examples of substitution in the $R_2$ phenyl group include halogen atoms and especially chlorine atoms and alkyl or alkoxy groups containing 1 to 4 carbon atoms and especially methyl or methoxy groups.

Particularly suitable $R_2$ groups are 2, 4 6-trichlorophenyl, 2,4-dichloro-6-methyl phenyl and 2,6-dichloro-4-methoxy phenyl.

Preferably $R_3$ comprises an alkanoyl amino group having at least 12 carbon atoms. A particularly suitable

group is the myristoyl amino group which comprises 14 carbon atoms. Another useful group $R_3$ is a ballasted sulphonylamino group, for example hexadecyl sulphonylamino. Yet another suitable $R_3$ ballasting group is a substituted alkanoyl amino group where the oil-solubilising substituent is an aryloxy group for example di-tertiary-aryl phenoxy acetyl. Another useful group $R_3$ is a ballasted succinamido group.

Ballasting groups of the above types are well known in the art.

The pyrazolone compounds of formula I may be prepared by routes known in the literature, and in particular by the methods described in British patent specification 2 086 597 which involves the reaction of a 4-equivalent pyrazolone coupler of formula II :-

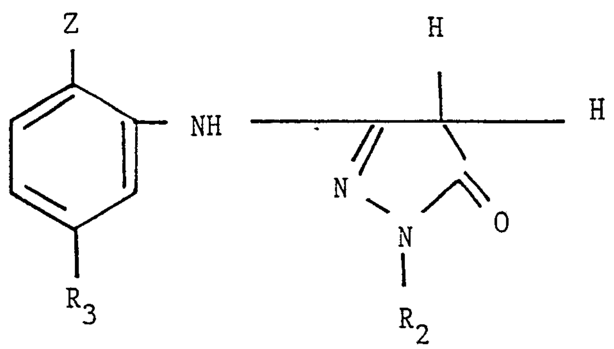

in which Z, $R_2$ and $R_3$ have the meanings defined above with the sulphenyl chloride prepared from a thiol of general formula III :-

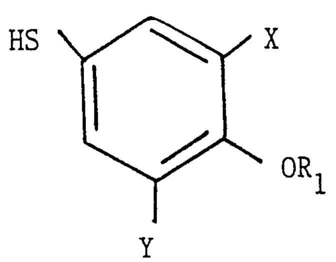

in which $R_1$, X, and Y have the meanings defined above.

The four equivalent couplers of formula II are known compounds, and the thiols of formula III may be prepared by known methods, as described for example, by J C Wardell in Chapter 4 of the book The Chemistry of the Thiol Group, edited by S Patai.

It is to be understood that the compounds of formula I are potentially tautomeric, and that other tautomers may be involved under some circumstances.

According to another aspect of the present invention there is provided silver halide photographic material which comprises in at least one layer at least one pyrazolone colour coupler of general formula I.

The photographic material may be the normal tripack colour photographic material comprising at least one blue light sensitive layer which comprises a yellow dye yielding colour coupler, at least one red light sensitive layer which comprises a cyan dye yielding colour coupler and at least one green light sensitive layer which comprises a magenta dye yielding colour coupler of formula I.

All yellow and cyan colour couplers known in the art can be used.

Preferably however the photographic material of this aspect of the present invention is a so-called chromogenic monochrome material in which there is present a plurality of colour couplers which form a dye image rather than a silver image, which dye image is an almost grainless image which can be used for enlarging purposes. Such material is described in European patent application 25775.

As described in European patent application 25775 there is present in the chromogenic monochrome material preferably a yellow colour coupler, a cyan colour coupler and a magenta colour coupler. Most preferably the chromogenic monochrome material comprises two silver halide emulsion layers each of which comprises a yellow colour coupler, a cyan colour coupler and a magenta colour coupler of formula I as hereinbefore set forth.

These colour couplers are preferably dispersed in a solvent as described in U.S. patent specification 3676137.

An organic solvent is preferably employed which is capable of dissolving the coupler, scarcely soluble in water, having a high boiling point and which remains together with the coupler in the colour photographic light-sensitive material containing the emulsion. Suitable solvents include substituted hydrocarbons, carboxylic acid esters, carboxylic acid amides, phosphoric acid esters and ethers.

Specific examples include di-n-butylphthalate, di-iso-octyl azelate, di-n-butyl sebacate, tricresyl phosphate, tri-n-hexyl phosphate, N,N-diethylcaprylamide, butyl-m-pentadecylphenyl ether and chlorinated paraffins.

In addition to the high boiling solvent, there is sometimes used an auxiliary solvent for dissolving the coupler, which solvent can be removed during the production of the photographic light-sensitive material. Specific examples of auxiliary solvents include propylene carbonate, ethyl acetate, butyl acetate, cyclohexanol, tetrahydrofuran and cyclohexanone.

Sometimes a surface active agent is used as an aid to disperse the oil soluble coupler of the incorporated type in the hydrophilic polymer of the photographic emulsions, and particularly, an anionic surface active agent such as sodium cetylsulphate, sodium p-dodecylbenzenesulphonate, sodium nonylnaphthalenesulponate or sodium di(2-ethylhexyl)-sulphosuccinate, or nonionic surface active agents such as sorbitan sesquioleic acid esters or sorbitan monolauric acid esters.

To disperse an oil-soluble coupler, an emulsifying homogenizer, a colloid mill or an ultrasonic emulsifying apparatus may be employed.

The photographic material of the present invention may be coated on any of the usual film bases including cellulose triacetate, cellulose acetate-butyrate and subbed and oriented polyethylene terephthalate. Preferably the silver halide used is an iodobromide silver halide having a halide ratio of from 1.5 to 10% of iodide to bromide.

Preferably the silver halide is panchromatically sensitized using both at least one green optical sensitizing dye and at least one red optical sensitizing dye.

The silver halide emulsion may be chemically sensitized by sulphur and/or gold sensitizers and also by polyethylene oxide compounds or by other sensitizing agents used to sensitize high speed camera film emulsions.

The silver halide emulsion may be stabilized by the presence of stabilizing compounds used to stabilize such emulsions, such as tetrazaindene compounds and mercaptotretazole compounds.

The binder for the silver halide crystals in the silver halide emulsion is preferably gelatin but so-called gelatin extenders may be present such as acrylamides and polyvinyl alcohol. Latex polymers may also be present such as latex polymers derived from alkyl acrylates and methacrylates.

The binder may be hardened using any of the well known hardeners such as formaldehyde, glyoxal, vinyl sulphones and triazine derivatives.

Conventional chromogenic processing baths can be used to develop the monochromatic material. The developer bath as a rule contains a developer substance of the p-phenylenediamine type, a development retarder, such as potassium bromide, an antioxidant, such as sodium sulphite, and a base, for example an alkali metal hydroxide or alkali metal carbonate.

Furthermore, the developing bath can contain a conventional antifogging agent and complex-forming agent.

The following specific compounds of general formula IV are of particular use in the present invention :-

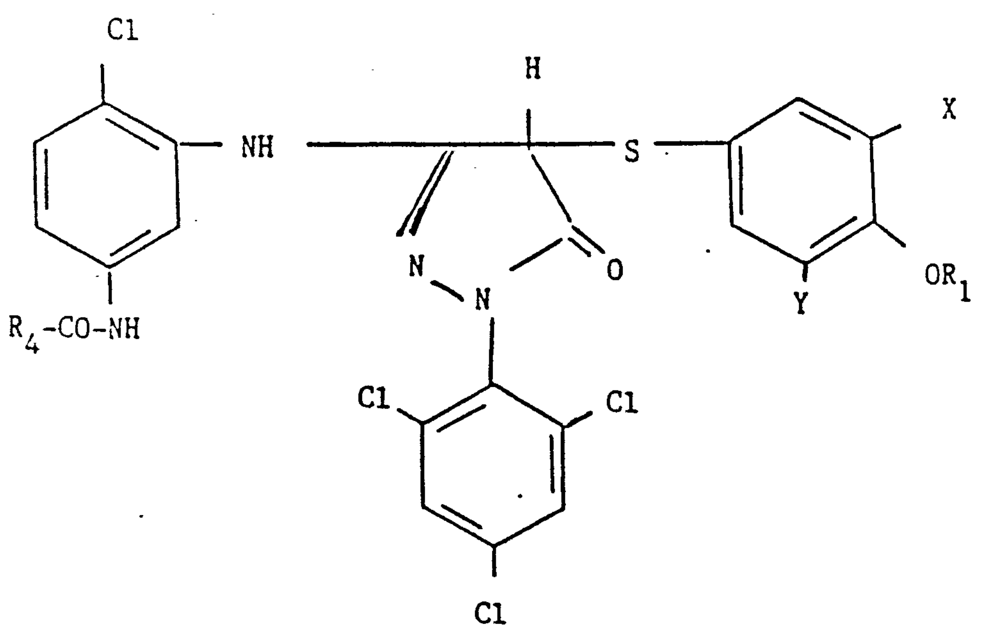

| COMPOUND | | | $R_4$ | Y |
|---|---|---|---|---|
| <u>A</u> | Cl | $n-C_8H_{17}$ | $n-C_{13}H_{27}$ | H |
| <u>B</u> | Br | $n-C_8H_{17}$ | $n-C_{13}H_{27}$ | H |
| <u>C</u> | Cl | $n-C_{10}H_{21}$ | $n-C_{13}H_{27}$ | H |
| <u>D</u> | Cl | $n-C_{12}H_{25}$ | $n-C_{13}H_{27}$ | H |
| <u>E</u> | Cl | $n-C_8H_{17}$ | $n-C_{13}H_{27}$ | $CH_3$ |
| <u>F</u> | F | $n-C_8H_{17}$ | $n-C_{13}H_{27}$ | H |
| <u>G</u> | Cl | $n-C_8H_{17}$ | $n-C_{15}H_{31}$ | H |
| <u>H</u> | Cl | $n-C_{12}H_{25}$ | $n-C_{13}H_{27}$ | Cl |
| I | Cl | 2-Ethylhexyl | $n-C_{13}H_{27}$ | H |
| J | Cl | 3,7-Dimethyloctyl | $n-C_{13}H_{27}$ | H |
| K | Cl | $n-C_8H_{17}$ | $CH_2O$ 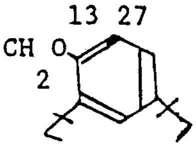 | H |

and compound L of the formula

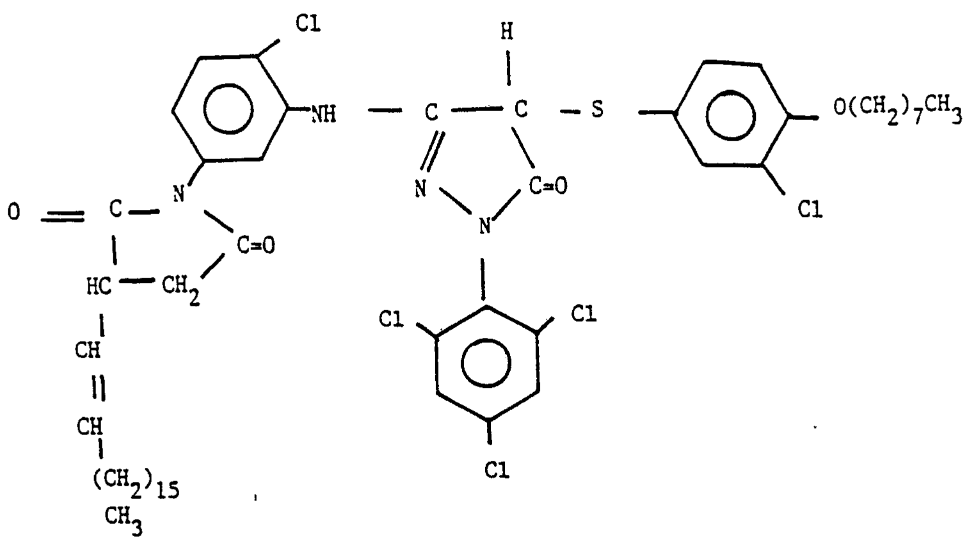

<u>EXAMPLE 1</u>

<u>Example of synthesis</u>

Synthesis of Coupler A using the following synthetic route :-

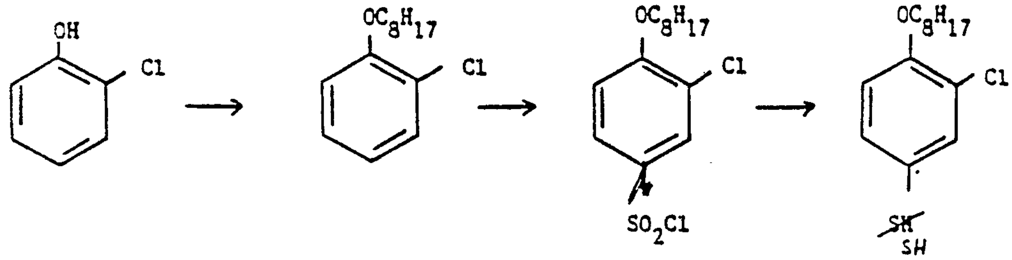

(a) (b) (c)

Intermediates

Synthesis of intermediate (a)

Sodium hydroxide (40g) in methylated spirit (500 ml) was heated under reflux to dissolve, and cooled somewhat before ortho chlorophenol (128.5g) in methylated spirit (125 ml) was added over a few minutes. 1-Bromo-octane (193 g) in methylated spirit (125 ml) was then added and the reaction heated under reflux for 7.5 hours, during which time a precipitate of sodium bromide appeared. After allowing to cool overnight, water (500 ml) and light petroleum (bp 60 - 80°C) (500 ml) was added, the organic layer was separated and washed successively with water, dilute sodium carbonate solution, water, dilute hydrochloric acid, and water, and dried over sodium sulphate before the solvent was evaporated leaving intermediate (a) as an oil, yield 236.6g.

Synthesis of intermediates (b) and (c)

Chlorosulphonic acid (64 ml) was added over 30 minutes to intermediate (a) (192.6g) in dichloromethane (400 ml) at 5-8°C, and was then stirred for 90 minutes at 5°C. The solvent was evaporated in vacuo, and the residual red oil taken up in acetonitrile (80 ml) and dimethylacetamide (240 ml), during which the temperature was allowed to rise to 40°C. Phosphoryl chloride (148 ml) was then added over 25 minutes, during which the temperature fell to 30°C, and the reaction was then allowed to fall to room temperature overnight.

The product (intermediate b) and acetonitrile (100ml) was added to concentrated sulphuric acid (120 ml) in ice (480 g), and zinc dust (192 g) was slowly added such that the temperature remained below 98°C. The reaction was then heated at 85°C for 2 hours, allowed to cool somewhat, light petroleum (bp 60-80°C) added, and solids removed by filtration. The organic layer was separated, washed with water, dried over sodium sulphate, and evaporated to give intermediate (c) as a pale brown oil yield 176.1 g.

Synthesis of coupler A

Sulphuryl chloride (1.8 ml) in dichloromethane (5 ml) was added to the above thiol (intermediate c) (5.45g) in dichloromethane (15 ml) over about 7 minutes, and stirred at room temperature for a further 30 minutes. The solvent was evaporated, and the residual sulphenyl chloride taken up in dichloromethane (20 ml) and added to the 4-equivalent coupler 1-(2,4,6-trichlorophenyl)-3-(2-chloro-5-myristoylamino anilino)- pyrazolone (12.3g) in dichloromethane (50 ml) and triethylamine (11 ml). After 1 hours at room temperature the mixture was heated under reflux for 2 hours, and then allowed to cool. The product was washed twice with dilute hydrochloric acid, and then with water before being dried. After the solvent was evaporated the residue of coupler A slowly crystallised, and was recrystallised from ethyl acetate (100ml) and acetonitrile (50 ml), yield 9.65g (55%) Mp 205 -208°C.

The following couplers were also prepared using the same technique :-

| COUPLER | m.p. (°C) |
|---|---|
| B | 204.5 - 206 |
| C | 199 - 201 |
| D | 202 - 203 |
| E | 172.5 - 175.5 |
| F | 191 - 194 |
| G | 202 - 204.5 |
| H | 169 - 171 |
| I | 169 - 171 |
| J | 201.5 - 203 |
| K | oil |
| L | oil |

## EXAMPLE 2

Example of use

In the following examples, 2-equivalent magenta couplers M and N were used as control couplers for comparison with the couplers of the invention

Coupler M

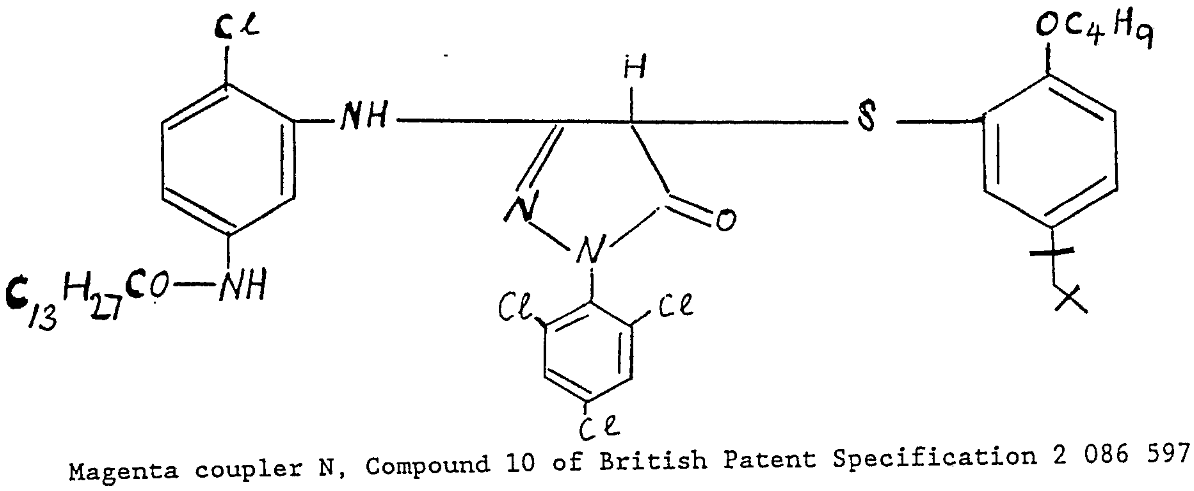

Magenta coupler N, Compound 10 of British Patent Specification 2 086 597

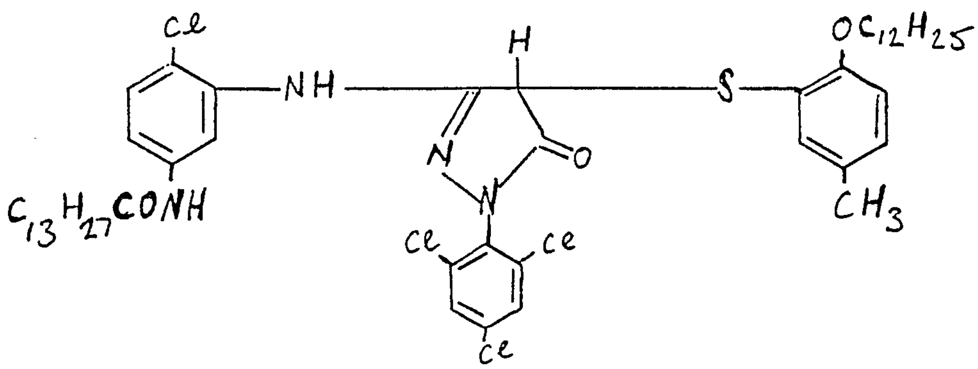

Cyan coupler O an naphtholic coupler

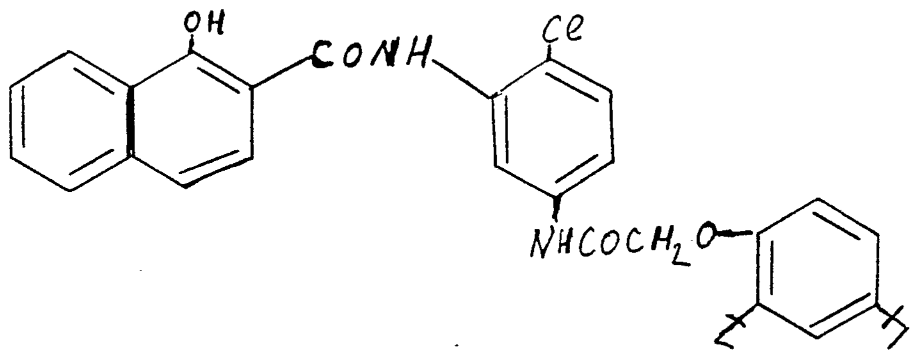

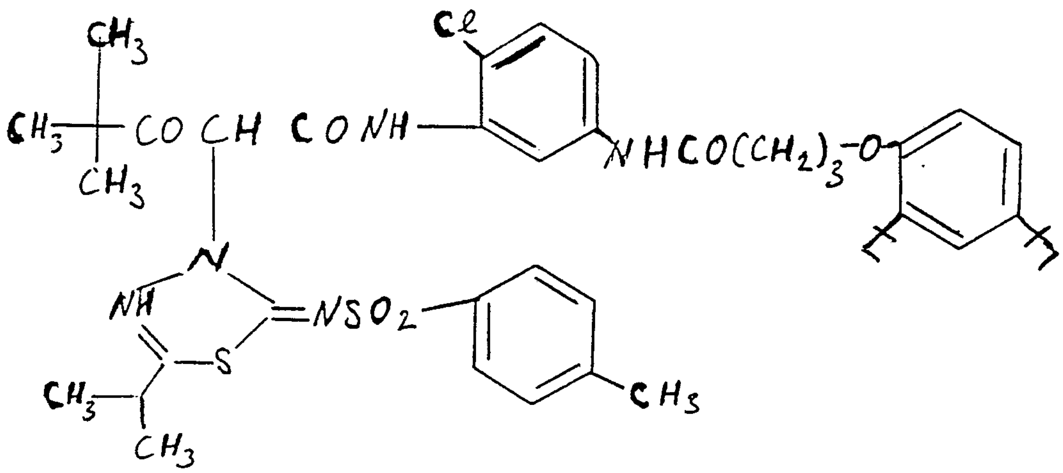

Coupler P, Yellow coupler

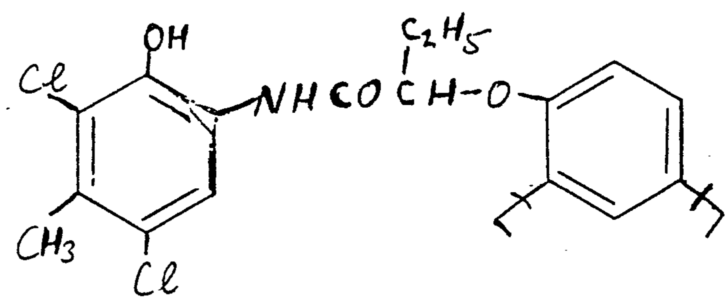

Coupler Q, phenolic cyan coupler.

These couplers were formulated into an emulsion as follows :

The following were heated under reflux until a clear solution was obtained :-

| | | |
|---|---|---|
| Yellow coupler P | 6.0 g | |
| Magenta coupler | 2.0 g | (A,B,C,E,F,M or N) |
| Cyan coupler Q | 1.5 g | |
| Cyan coupler O | 0.5 g | |
| Triaryl phosphate oil | 7.5 g | |
| Ethylacetate | 5.0 g | |

This solution was cooled to 50°C, and 0.5 g wetting agent added, and then rapidly mixed with a solution of

| | |
|---|---|
| 10% gelatin solution | 40 g |
| Water | 27 g |
| Wetting agent (Nekal BX 10%) | 10 g |

and then treated with an ultrasonic mixer for 4 minutes to give a pure white dispersion with a particle size of less than 300nm.

This dispersion was mixed with a conventional dyed silver halide emulsion and coated to give a silver coating weight of 50 $mg/dm^2$.

Samples of these coatings were then exposed for 1/30 seconds to a daylight wedge exposure of 250 lux and processed using standard colour processing conditions.

The colour processing employed was as follows:-

1. Colour developing 3 1/4 minutes

developer bath :

| | |
|---|---|
| potassium carbonate | 37.5 g |
| sodium metabisulphite (anhydrous) | 4.25 g |
| potassium iodide | 2.0 mg |
| sodium bromide | 1.3 g |
| Hydroxylamine sulphate | 2.0 g |
| 4-(N-ethyl-N-B-hydroxyethylamino)-2-methylaniline sulphate | 4.75 g |
| Water to make up to | 1 litre |

2. Bleaching 6 $\frac{1}{2}$ minutes

bleaching bath :

| | |
|---|---|
| ammonium bromide | 150 g |
| ammonium salt of the iron-III-complex of ethylenediamine tetra-acetic acid | 175 ml |
| acetic acid (glacial acetic acid) | 10.5 ml |
| sodium nitrate | 35 g |
| water to make up to | 1 litre |

3. Washing 3 1/4 minutes
4. Fixing 6 1/2 minutes

fixing bath

| | |
|---|---|
| ammonium thiosulphate (50% aqueous) | 16.2 ml |
| diethylenetriaminepenta-acetic acid | 1.25 g |
| sodium metabisulphite (anhydrous) | 12.4 g |
| sodium hydroxide | 2.4 g |
| water to make up to | 1 litre |

5. Washing 3 1/2 minutes

The density and contrast of the processed samples were measured immediately after processing. The densities and contrasts of the magenta and cyan dye images were measured.

The processed samples were then incubated for 24 hours. This incubation is sufficient to complete the elimination step of the leaving group of the two-equivalent magenta coupler. The density and contrast of the incubated samples were then measured again. An increase in density and contrast of the magenta dye image after incubation is a disadvantage, since it reveals that the magenta dye image was not fully formed after the standard processing conditions.

From the results in the accompanying table it will be seen that the control couplers M and N show severe increases in density of the magenta dye image after incubation, and also that the control coupler M shows a decrease in density of the cyan dye image on incubating, and that these disadvantages are practically eliminated with the inventive couplers A, B, C, E and F.

The visible absorption spectram from 350 to 850 nm before and after incubation using control compound N in the emulsion are shown in the accompanying figure 1 and using colour coupler J of the present invention in the emulsion are shown in figure 2.

In both figures the full line is before incubation and the broken line is after incubation.

As shown in these figures there is almost no alteration in density after incubation when using colour coupler J but there is a marked increase in the magenta dye peak density around 550 nm when using compound N.

TABLE

| COUPLER | TO GREEN LIGHT (i.e. Magenta dye) | | | | TO RED LIGHT (i.e. Cyan dye) | | | |
|---|---|---|---|---|---|---|---|---|
| | Before incubation | | Change on incubation | | Before incubation | | Change on incubation | |
| | Dmax | Contrast | Dmax | Contrast | Dmax | Contrast | Dmax | Contrast |
| M (control) | 1.24 | 0.73 | +0.73 | +0.53 | 2.01 | 1.08 | -0.10 | -0.06 |
| N (control) | 1.86 | 0.88 | +0.24 | +0.11 | 2.02 | 0.85 | -0.05 | -0.06 |
| A | 1.86 | 0.69 | +0.05 | +0.02 | 1.68 | 0.59 | -0.05 | -0.01 |
| B | 1.89 | 0.72 | +0.02 | +0.01 | 1.74 | 0.65 | -0.07 | -0.04 |
| C | 1.85 | 0.70 | +0.04 | +0.02 | 1.68 | 0.58 | -0.07 | -0.01 |
| E | 1.71 | 0.61 | +0.04 | +0.02 | 1.61 | 0.58 | -0.07 | -0.03 |
| F | 1.91 | 0.71 | +0.02 | 0 | 1.69 | 0.60 | -0.08 | -0.03 |

## Claims

1. A pyrazolone compound of general formula I :

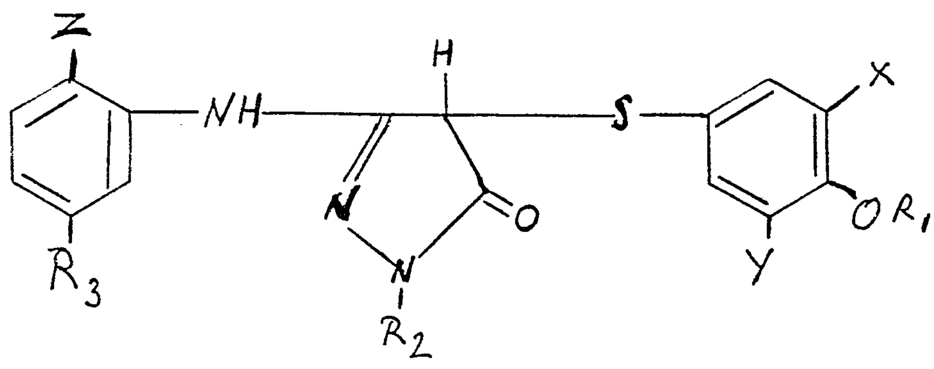

wherein X is a halogen atom, Y and Z are each hydrogen, halogen, alkyl, aryl, alkoxy, aryloxy, acylamino or sulphonamido, $R_1$ is a alkyl group having at least 6 carbon atoms, $R_2$ is substituted phenyl and $R_3$ is a ballasting group which renders the colour coupler oil-soluble and which is attached to the substituted phenyl ring by a nitrogen atom.

2. A pyrazolone compound according to claim 1 wherein $R_1$ is a straight or branched chain or cyclic alkyl group having 8 to 12 carbon atoms.

3. A pyrazolone compound according to either claim 1 wherein X is a chlorine atom.

4. A pyrazolone compound according to claim 1 wherein, Z is a chlorine atom.

5. A pyrazolone compound according to claim 1 wherein Y is a hydrogen atom or a methyl group.

6. A pyrazolone compound according to of claim 1 wherein, the $R_2$ phenyl group is substituted in the 2, 4 and 6 positions.

7. A pyrazolone compound according to claims 1 wherein, $R_3$ comprises an alkanoyl amino group having at least 12 carbon atoms.

8. A pyrazolone compound according to claim 7 where in the $R_3$ is a myristoyl amino group.

9. A process for the preparation of a pyrazolone compound according to claim 1, which comprises reacting a pyrazolone A wherein $Z_1$, $R_2$ and $R_3$ have the meanings assigned to them in claim 1, with the sulphenyl chloride prepared from a thiol of the formula B

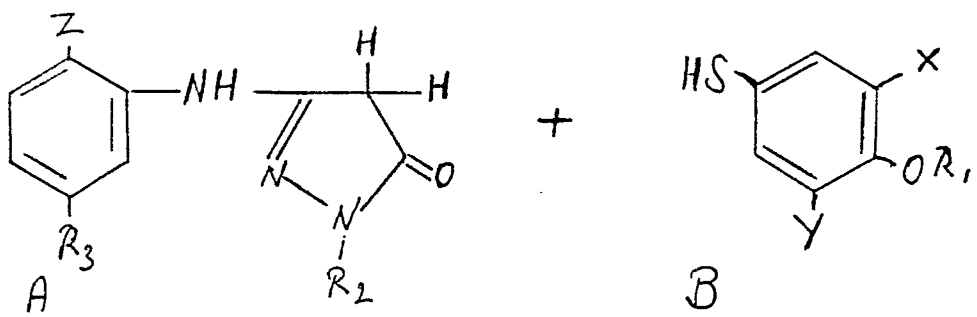

A + B

wherein $X_1$ and $R_1$ have the meanings assigned to them in claim 1.

10. Silver halide photographic material at least one layer which comprises at least one pyrazolone colour coupler as claimed in claim 1.

11. Silver halide photographic material according to claim 10 which comprises at least one blue light sensitive layer which comprises a yellow dye yielding colour coupler, at least one red light sensitive layer which comprises a cyan dye yielding colour coupler and at least one green light sensitive layer which comprises a magenta dye yielding colour coupler which is a pyrazolone colour coupler as claimed in claims 1.

12. Silver halide photographic material according to claim 10, whcih comprises at least one silver halide layer which contains at least one magenta dye yielding pyrazolone colour coupler as defined in claim 1, at least one a yellow dye yielding colour coupler and at least one cyan dye yielding colour coupler, the colour couplers being dissolved in a high boiling point organic solvent.

13. Photographic silver halide material according to claim 10 wherein the silver halide used is an iodobromide silver halide having a halide ratio of from 1.5 to 10% by weight of iodide to bromide.

14. Photographic silver halide material according to claim 12 wherein the silver halide is panchromatically sensitized using both at least one green optical sensitizing dye and at least one red optical sensitizing dye.

15. A thiol of the formula III

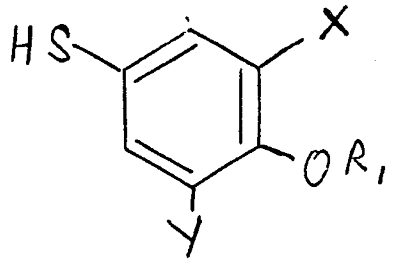

wherein, X, Y and $R_1$ have the meanings assined to in claim 1.

16. A thiol according to claim 15 wherein X is selected from Cl, Br and F, Y is selected from H, Cl and $CH_3$ and $R_1$ is selected from n-$C_8H_{17}$, , n-$C_{10}$ $H_{21}$, n-$C_{12}H_{25}$, 2-ethylhexyl, and 3,7-dimethyloctyl.

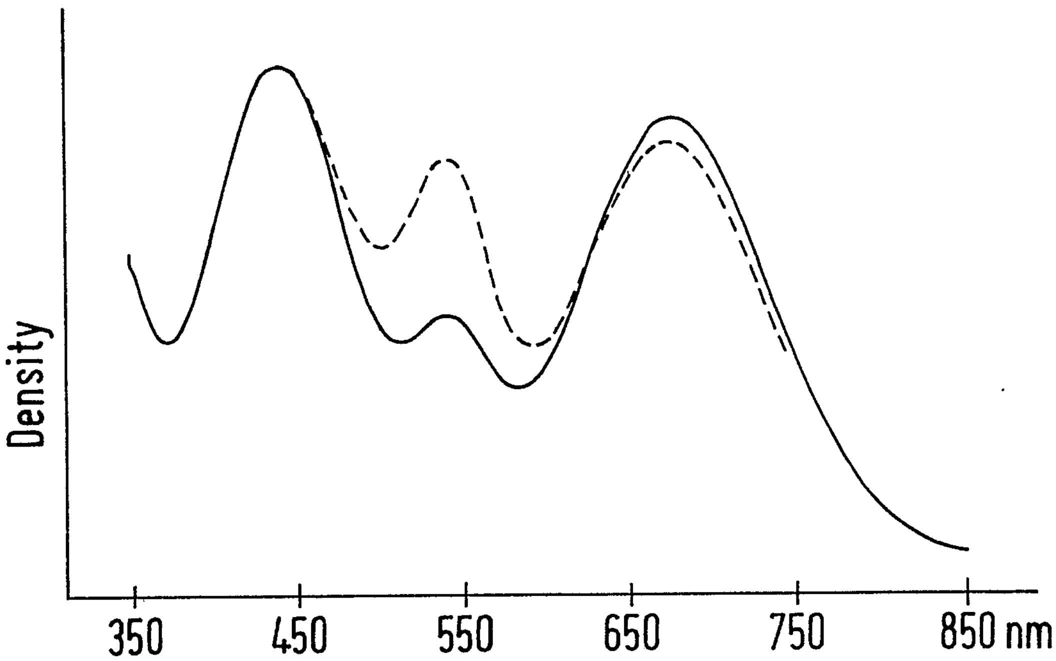
Density
350
450
550
650
750
850 nm

FIG.1

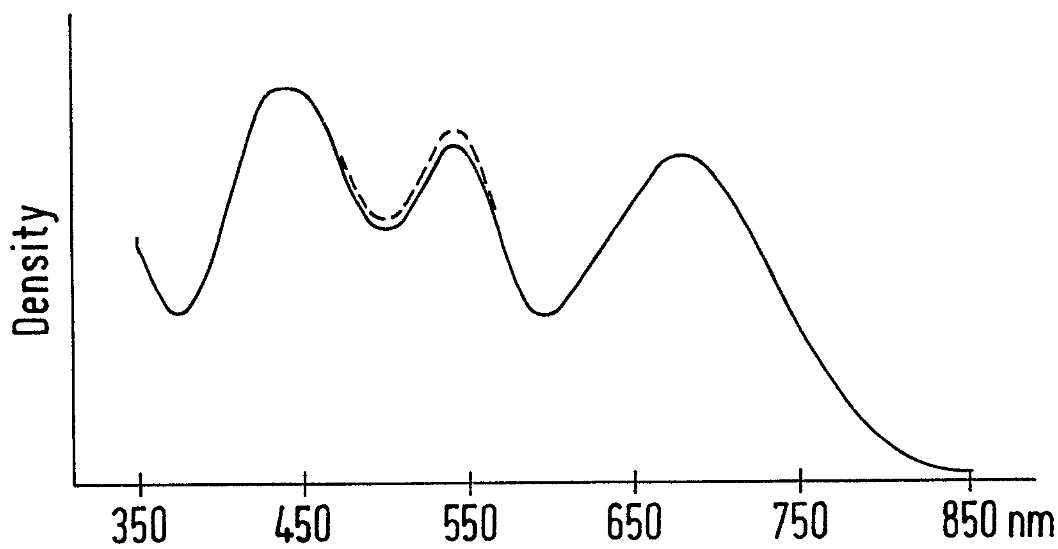
Density
350
450
550
650
750
850 nm

FIG.2